(19) **Europäisches Patentamt European Patent Office Office européen des brevets**

(11) **EP 1 611 385 B1**

(12) **EUROPEAN PATENT SPECIFICATION**

(45) Date of publication and mention of the grant of the patent:
**26.12.2007 Bulletin 2007/52**

(51) Int Cl.:
*F16L 11/12* (2006.01)     *A61F 2/06* (2006.01)
*F16L 9/00* (2006.01)     *E21B 17/18* (2006.01)

(21) Application number: **04721591.8**

(22) Date of filing: **18.03.2004**

(86) International application number:
**PCT/GB2004/001170**

(87) International publication number:
**WO 2004/083706 (30.09.2004 Gazette 2004/40)**

(54) **TUBING AND PIPING FOR MULTIPHASE FLOW**

SCHLÄUCHE UND ROHRE FÜR MEHRPHASENSTRÖMUNG

TUYAUTERIE ET CANALISATION POUR ECOULEMENT POLYPHASIQUE

(84) Designated Contracting States:
**AT BE BG CH CY CZ DE DK EE ES FI FR GB GR HU IE IT LI LU MC NL PL PT RO SE SI SK TR**

(30) Priority: **18.03.2003 GB 0306179**
          **18.03.2003 GB 0306180**
          **18.03.2003 GB 0306176**

(43) Date of publication of application:
**04.01.2006 Bulletin 2006/01**

(73) Proprietor: **IMPERIAL COLLEGE INNOVATIONS LIMITED**
**London SW7 2QA (GB)**

(72) Inventors:
• **CARO, Colin, Gerald;**
**Imperial College**
**London SW7 2AZ (GB)**

• **WATKINS, Nicholas, V.Imperial College**
**London SW7 2AZ (GB)**
• **BIRCH, Philip, LloydOld Pockford Cottage**
**Chiddingfold GU8 4XU (GB)**
• **YACOUB, Magdi Imperial College**
**London SW7 2AZ (GB)**

(74) Representative: **Stevens, Jason Paul**
**Frank B. Dehn & Co.**
**St Bride's House**
**10 Salisbury Square**
**London EC4Y 8JD (GB)**

(56) References cited:
**WO-A-00/38591          US-A- 5 553 976**
**US-A- 6 039 754          US-B1- 6 343 516**

Printed by Jouve, 75001 PARIS (FR)

## Description

This invention relates to tubing and piping for use in multiphase flow.

Multiphase flow is of course well known, and occurs when the flow in a tube or pipe is not made up of a single homogenous fluid. Examples of multiphase flow are gas/liquid, liquid/solid (such as suspensions and slurries), gas/solid (powders entrained in air), two immiscible liquids (such as oil and water), liquids at different temperatures, and so on.

Multiphase flow can lead to serious problems. A principal problem is that the phases are often of differing densities. For example, gases (being of a lower density than liquid) can accumulate in the upper part of a substantially horizontal pipe carrying a gas/liquid mixture, and this can cause problems if the pipe carrying the fluids is not perfectly horizontal along its length. If there are undulations along the length of the pipe, then gas can accumulate in the upper parts of the undulations and lead to airlocks. Similarly, the denser of two immiscible liquids can collect in the lower parts of a pipe, and lead to similar locks.

These problems can be particularly severe in the hydrocarbon (oil and gas) extraction industry. In this industry, it has become increasingly common to drill a well vertically, and then to navigate the drill to a generally horizontal orientation. A typical well may penetrate a few kilometres vertically down into the earth and then have a horizontal portion of many hundreds of metres. This type of well drilling enables a single surface location to be used to access reservoir formations over a wide area, rather than only immediately below the surface location. In addition, the horizontal portion of the well may be used to access horizontally spaced compartments of a hydrocarbon reservoir.

It has also become increasingly common to extract hydrocarbons from deep, high pressure/high temperature reservoirs, where low molecular weight hydrocarbons exist in a liquid form called gas condensate. Owing to their high cost, such reservoirs adapt themselves to be drained by horizontal production wells.

A typical reservoir may contain liquid hydrocarbon sitting over water. The horizontal portion of the well extends along the liquid hydrocarbon layer. Fluids move from that layer into the well bore, via wall perforations provided at selected points, where they enter a lower pressure regime. The liquid hydrocarbon segregates to gas and liquid hydrocarbon, and water is often included in the mixture entering the well. The gas phase may predominate, with secondary phases of liquid hydrocarbon and water, or the liquid hydrocarbon may predominate, with secondary phases of gas and water. Either way, the well has to transport a multi-phase fluid, which will normally consist of gas and two immiscible liquids.

In practice, the horizontal well portion is rarely exactly horizontal over its length. During the initial drilling process a generally undulating horizontal well tends to be created. This results, in effect, in the formation of gentle U-bends in the horizontal well portion. Viewing the well from the outside, these may take the form of upwardly convex U-bends and upwardly concave U-bends. As the multi-phase fluid flows along the well it is not uncommon for gravity separation of the phases to occur. Water gathers at the bottom of any upwardly concave U-bends, whilst gas may collect at the top of any upwardly convex U-bends.

If the water fills a U-bend the flow is occluded. Well production ceases when it builds up too much dense fluid. The accumulation of gas can lead to terrain-induced slugging. Slugging occurs when gas bubbles collect on the walls of the pipe to such an extent that they block the flow entirely. Liquid approaching this blockage will tend to raise the pressure of the gas, and when the pressure reaches a certain point the blockage will suddenly shift. This sudden restart of flow (or "explosion") causes large shock loads on the pipe, and also on any downstream piping or equipment, which can cause serious damage.

A submersible pump can be deployed into the well to extract the water. However, this takes time, and production can be halted for several days or even longer. Furthermore, as the hydrocarbon reservoir is drained the water content in the fluid may increase, leading to more frequent occurrences of well occlusion. Whilst the process is most common in horizontal wells, it can present a problem in any multiphase well. Also, the use of a submersible pump does not solve the problem of terrain-induced slugging.

Another problem associated with water accumulation is the precipitation of minerals in the well which may also lead to occlusion or choking. Further, the presence of water can lead to turbulence, which may lead to stagnant or dead areas in the pipe. Precipitation (of minerals or of hydrocarbons) and sedimentation are more likely to occur in these areas.

A further problem in multiphase production wells occurs in the low-temperature and pressure upper reaches of the well, particularly in submarine risers connecting the sea-floor well-head to the production vessel or platform. Under these conditions, gas can form large bubbles which can lead to severe slugging. In addition, large bubbles will significantly increase pressure-loss within the well, thus inhibiting production.

A further specific situation where the formation of airlocks and so on would be extremely undesirable is in the tubing used during heart operations.

During open-heart surgery, the heart of the patient is stopped. In order to maintain circulation, blood is normally withdrawn from the right atrium, passed through a pump and an oxygenation unit, and then returned to the aorta for circulation around the patient's body.

Air can be entrained into the blood as it is withdrawn from the patient's heart, and can form bubbles in the tubing leading from the patient to the pump and the oxygenation unit. Bubbles of oxygen can also form in the blood during the oxygenation process.

Further, there is a trend in general surgery (not necessarily open-heart) to reduce the amount of donated blood used. The patient's own blood is recirculated, and the collection device used to collect the patient's blood can easily entrain air, which will form bubbles.

Obviously, these bubbles must be removed from the blood before it is returned to the patient, and bubble traps are routinely provided in the tubing to allow this removal.

However, there is a known problem regarding the bubbles, in that they can accumulate in the tubing connecting the patient, the pump and the oxygenation unit. Although the bubbles can be loosened from the tubing by tapping the tubing, an unnoticed build-up of bubbles can lead to blockages, and (if not dealt with) interruption of the blood supply, which can have extremely serious consequences.

According to a first aspect of the invention, there is provided tubing or piping which is:

> well production tubing, such as risers, flow lines, and subsea and surface tubing; or
> blood flow tubing; or
> transport tubing or piping for transporting suspensions and/or slurries; or
> transport tubing or piping for transporting a suspension of powder in gas; the tubing or piping having features which induce swirl flow in a multiphase flow, in such a manner that denser components of the multiphase flow tend to the outer wall of the tubing or piping, and less dense components of the multiphase flow tend to the centre of the tubing or piping, as fluids flow along the pipe, wherein the centre line of the tubing or piping follows a substantially helical path; characterized in that the amplitude of the helix is less than or equal to one half of the internal diameter of the tubing or piping.

It has been found experimentally that swirl flow provides considerable advantages in the context of multiphase flow. In multiphase swirl flow, it has been found that the lighter fractions of the flow (such as gases and less dense liquids) tend to flow along the centre of the pipe, while the heavier fractions of the flow (denser liquids) flow along the walls of the pipe in a generally helical path. It is believed that this arises from the centrifugal effect of the swirling flow. As a result, there is much less tendency for lighter or heavier fractions to separate out under gravity.

Swirl flow provides considerable advantages in the context of multiphase flow. As there is less tendency for lighter or heavier fractions to separate out under gravity, the risk of airlocks occurring is greatly reduced. Similarly, denser liquids will not collect in the lower parts of the pipe, and so there is less risk of flow disruption arising in this way.

These advantages will be discussed further with reference to well production tubing. As mentioned above, the horizontal portion of known well production tubing may undulate horizontally as well as vertically. The curves in the well so created have such a low curvature as to have a negligible effect on the nature of fluid flow along the well. The flow (providing of course that it is not occluded) may therefore be considered as having the characteristics of flow along a straight pipe. The flow will normally be turbulent, although in accordance with known pipeline hydraulics, a thin laminar layer is present in proximity to a solid boundary, i.e. the tubing inner wall. For slower flow speeds, the flow may be laminar. In both cases, the axial velocity profile in straight tubing flow has a maximum at the centre of the tubing, with slower velocities adjacent to the walls.

One effect of swirl flow is that the axial velocity profile of the flow across the tubing becomes more uniform or "blunter", with the speed of flow near the tubing wall being faster than it would be in similar flow in a straight well production tubing. The flow at the centre of the tubing is slower than would be the case in straight tubing. Because of the blunter velocity profile, fluid flowing in the tubing acts in the manner of a plunger. This will tend to reduce the accumulation of water or other dense fluids at low points of the tubing (upwardly concave U-bends) and the accumulation of gas at high points (upwardly convex U-bends).

A further major benefit of swirl flow is the promotion of mixing in a multiphase flow. In well production tubing, gas, liquid hydrocarbon and water will tend to mix, and so the tendency for accumulation of liquids along the tubing will be reduced. The better mixing and higher near-wall flow speeds will also reduce the opportunities for the sedimentation of solids to occur at low points along the well, or for minerals to precipitate.

This will also be of importance in higher portions of the well, where bubbles can coalesce. The mixing effects of swirl flow may enhance phase mixing and prevent large bubbles from forming. The promotion of swirl flow is of benefit in steep wells, e.g. vertical or 45° to the horizontal, and not just in horizontal well portions.

However, to the extent that the components of a multi-phase fluid flow do not mix, as the fluid flows axially along the tubing of this invention the denser components will tend to revolve around the tubing near the wall, with less dense components revolving nearer to the centre. This "centrifuge" phenomenon assists reduction of accumulation of e.g. water at low points of the tubing and reduction of accumulation of gases at high points.

All of these three factors (blunter velocity profile, improved mixing and "centrifuge" effect) are believed to contribute to improved flow characteristics with multiphase swirl flow.

The well production tubing as discussed here includes any multiphase transmission tubing. In the context of oil production, it includes inter alia the tubing below a well head, any surface flow lines, risers, and any tubing for transporting and/or processing multiphase petroleum.

Various means are known for inducing swirl flow along tubing or piping; WO 00/38591 suggests helical ridges or grooves in the tubing or piping wall, or guide vanes extending inwardly from the wall. However, this is not considered to be an optimum solution, since such devices may themselves form obstructions or create stagnant regions where material may accumulate. In addition, the ratio of the wetted perimeter to the cross-sectional area of the tubing would be increased by the provision of ridges, grooves, vanes etc. This may lead to increased flow resistance and pressure-loss, or conversely, to a reduction in flow for a given head.

Further, experiment has shown that unless the Reynolds number is very low, ridges, grooves or vanes of this type only have an effect on the flow near the wall of the pipe. It may be necessary to provide a long pipe in order to be sure that the flow swirls across the entire width of the pipe. Swirl in the centre of the pipe is only achieved through diffusional transfer of momentum from the flow at the wall of the pipe; the ridges, grooves or vanes do not facilitate mixing between fluid near the wall of the pipe and fluid at the centre of the pipe.

A further possibility would be for the tubing to have a non-circular cross-section which is twisted. However, a departure from circularity increases the ratio of the wetted perimeter to the cross-sectional area, which is undesirable. Further, this is not an efficient use of space.

Thus, in the invention, the centre line of the piping follows a substantially helical path.

In the above possible embodiments using grooves or ridges or non-circular sections, where the tubing is substantially straight, then the centre line of the tubing is also straight. The use of tubing with a helical centre line induces swirl and facilitates mixing between the fluid near the tubing wall and in the core in a better manner than where helical grooves or ridges are used in tubing with a straight centre line. In the case of tubing having a helical centre line, there is spatial reorganisation of vortical structures, which results in motion of the core or cores of the axial flow across the section of the tubing portion, promoting mixing across the cross section. The swirl inhibits the development of stagnation and flow separation regions and stabilises flows, and as mentioned above leads to the "centrifuge" effect.

Moreover, if the centre line of the tubing follows a substantially helical path, in accordance with the preferred embodiment, the tubing may have a circular cross-section and thus a small wetted perimeter to cross-sectional area ratio, and without obstructions to the flow. The tubing will still have the necessary characteristics to induce helical or swirl flow. There may however be circumstances in which it is desirable for tubing with a helical centre line to have a non-circular cross-section.

Well production tubing normally fits inside an outer casing. The tubing therefore has to occupy a swept width smaller than or equal to the internal diameter of the outer casing. In the case of the preferred helical tubing (i.e. tubing wherein the centre line follows a substantially helical path), if the helix is to have a large amplitude then the cross-sectional area available for fluid flow is correspondingly small. It is therefore preferred for the amplitude of the helix to be sufficiently large to induce swirl flow, but sufficiently small for the tubing to occupy as much as possible of the available cross-section. Optimization of the amplitude to meet the first of these criteria will depend on factors such as fluid viscosity, density and velocity.

In this specification, the amplitude of the helix refers to the extent of displacement from a mean position to a lateral extreme. So, in the case of tubing having a helical centre line, the amplitude is one half of the full lateral width of the helical centre line.

In the invention, the amplitude of the helix is less than or equal to one half of the internal diameter of the tubing. In such circumstances, there is a "line of sight" along the lumen of the tubing, unlike in the case of a corkscrew configuration where in effect the helix is wound around a core (either solid, or "virtual" with a core of air). It has been found that the flow at the line of sight generally has a swirl component, even though it could potentially follow a straight path.

For the purposes of this specification, the term "relative amplitude" of a helical tubing is regarded as the amplitude divided by the internal diameter. So, in the preferred embodiments in which the amplitude of the helical tubing is less than or equal to one half of the internal diameter of the tubing, this means that the relative amplitude is less than or equal to 0.5. Relative amplitudes less than or equal to 0.45, 0.40, 0.35, 0.30, 0.25, 0.20, 0.15, 0.1 or 0.05 may be preferred. Smaller relative amplitudes provide a better use of the available lateral space, i.e in the case of a helical tubing in a cylindrical outer casing there will be less unused space between the tubing and the outer casing. Smaller relative amplitudes also result in a wider "line of sight", providing more space for the insertion of pressure gauges or other equipment along the lumen of the tubing. With higher Reynolds numbers, smaller relative amplitudes may be used whilst swirl flow is induced to a satisfactory extent. This will generally mean that for a given tubing internal diameter where there is a high flow rate then a low relative amplitude can be used whilst being sufficient to induce swirl flow.

The angle of the helix is also a relevant factor in balancing the space constraints on a well production string with the desirability of having a large cross-sectional area available for flow. The helix angle is preferably less than or equal to 65°, more preferably less than or equal to 55°, 45°, 35°, 25°, 20°, 15°, 10° or 5°. As with relative amplitudes, the helix angle may be optimized according to the conditions: viscosity, density and velocity of fluid.

Generally speaking, for higher Reynolds numbers the helix angle may be smaller whilst satisfactory swirl flow is achieved, whilst with lower Reynolds numbers a higher helix angle will be required to produce satisfactory swirl. The use of higher helix angles for faster flows (higher Reynolds numbers) will generally be undesirable, as there may be near wall pockets of stagnant fluid. Therefore, for a given Reynolds number (or range of Reynolds numbers), the helix angle will preferably be chosen to be as low as possible to produce satisfactory swirl. In certain embodiments, the helix angle is less than 20°.

In general, the tubing will have a plurality of turns of the helix. Repeated turns of the helix along the tubing will tend to ensure that the swirl flow is maintained. However, even if a straight portion of pipe is provided downstream of a helical swirl-inducing section, it takes some distance for the swirl flow to die away, and so as an alternative to forming the entire pipe as a helical portion, it would be possible to provide a number of separate lengths of helical tubing or piping along the length of the pipe. These sections would then act as "repeaters". Each portion will induce swirl flow in the fluid passing through it; however, this swirl flow will tend to die away as the fluid passes along the straight pipe. Providing a number of "repeaters" allows the swirl flow to be re-established, with its concomitant benefits.

Similarly, helical portions can be provided before pipe fittings (such as elbow bends, T- or Y-junctions, valves and the like), so that swirl flow is established before the flow reaches these fittings.

Lengths of tubing will normally be made with substantially the same relative amplitude and helix angle along the length. There may be small variations when the tubing is deployed or in use, caused by elongation or contraction of the tubing due to tensile loading or caused by torsional loading. However, there may be circumstances in which the tubing has a variable helix angle and/or relative amplitude, either to suit space constraints or to optimise the flow conditions. For reasons of manufacturing simplicity, it will be preferred for the tubing to have a substantially constant cross-sectional area along its length. Again, there may be variations in use caused by loading on the tubing.

Similarly, considerable advantages can be achieved by forming the tubing used in machinery for heart operations as discussed above such that the fluid flowing in the tube flows in a swirl flow. The centrifuge effect means that any air or oxygen bubbles in the blood will tend to stay near the centre of the tubing, rather than accumulating at higher points of the tubing and leading to possible blockages. The bubbles will thus be carried along the tubing, and can be removed at bubble traps as discussed above.

Preferred embodiments of the invention will now be described by way of example and with reference to the accompanying drawings, in which:

Figure 1 is a schematic view of a long-reach horizontal well for hydrocarbon extraction, in accordance with the prior art;

Figure 2 is an enlarged view of part of the well of Figure 1;

Figure 3 is a view similar to Figure 2 but showing the use of tubing in a well in accordance with the invention;

Figure 4 is an elevation view showing tubing used in an experiment and designed to induce swirl flow in accordance with the invention; and

Figure 5 is a view similar to Figure 4 but showing another experiment.

Although the following description concentrates on the use of multiphase swirl flow in the context of hydrocarbon extraction, it will be appreciated that the advantages provided by multiphase swirl flow can be obtained in many other situations where multiphase flow occurs.

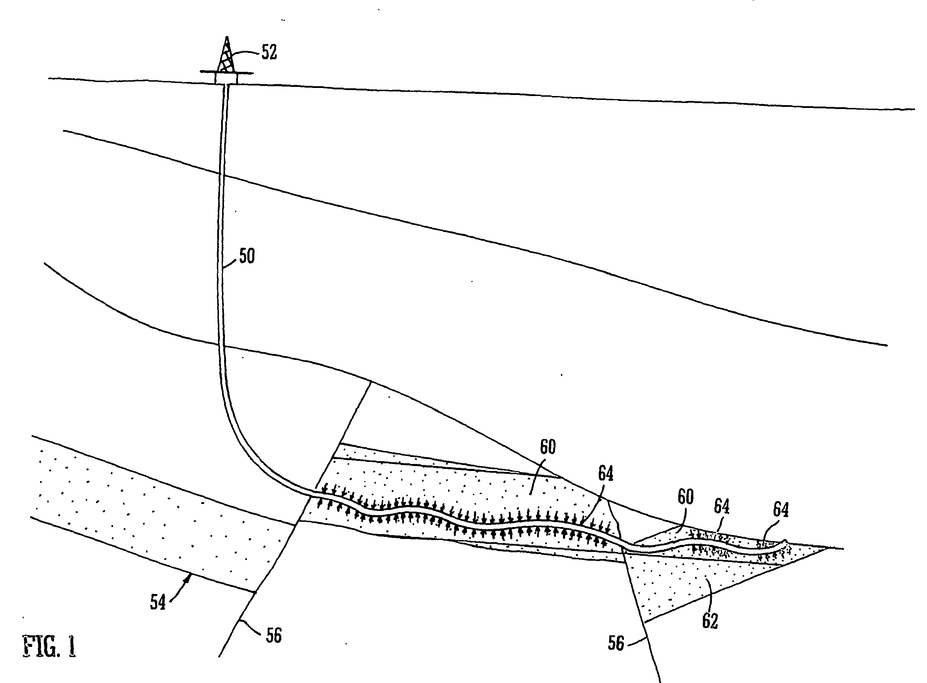

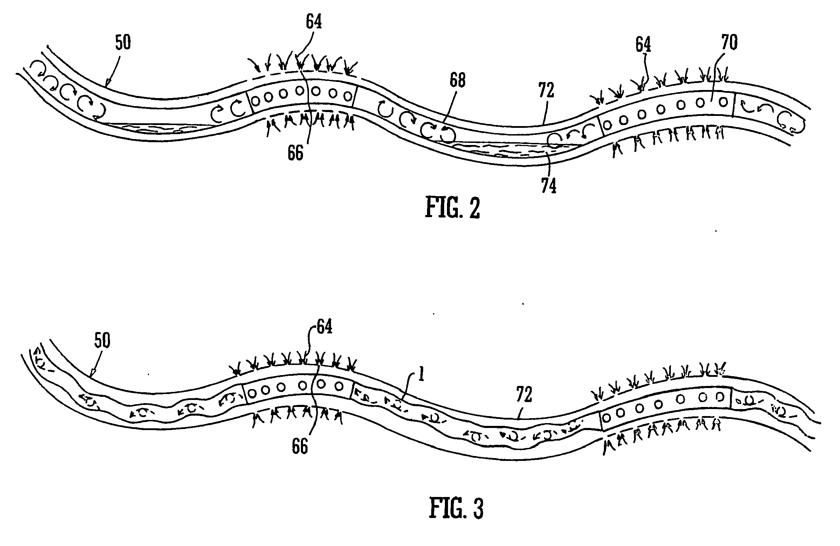

Figures 1 and 2 show the use of a long-reach horizontal well for hydrocarbon extraction, in accordance with a known method. A well production string 50 penetrates vertically into the ground from a well head 52 and at the required depth bends round to a generally horizontal orientation. The formation into which the well string is drilled includes a reservoir formation 54 separated into different zones by faults 56. The reservoir formation includes a liquid hydrocarbon layer 60, sitting on a water layer 62.

The well production string 50 includes sections formed with perforations 66 (see Figure 2) allowing entry of fluids into the well production string in the direction shown by arrows 64.

A known process for drilling such a well is as follows. A first portion is drilled to a specific depth and a first outer casing section is run down the drilling and cemented into place. The next portion of the well is drilled and another casing section is fed down the previously installed section and this is also cemented into place. The process continues, such that the diameters of successive outer casing sections decrease as the length of the well increases. Eventually the desired total length of the well is drilled and lined by outer casing sections.

Tubing 68, with perforating guns 70 provided at appropriate points according to the geology of the site, is inserted down the well. The perforating guns are fired, thereby creating the perforations 66 through the outer casing 72. This allows liquid hydrocarbon to pass from the reservoir 60 via the perforations 66 and into the well production string 50. The fluid in the well usually consists of a mixture of gas, oil and water. The multiphase fluid flows along the well production string 50 towards the surface. As seen in Figure 2, the horizontal portion of the well is not completely horizontal and has a series of gentle U-bends, both upwardly concave and upwardly convex.

Figure 2 shows a pool of water 74 which has collected in an upwardly concave U-bend. Eventually, this will fill the U-bend and cause a blockage which occludes the flow along the well.

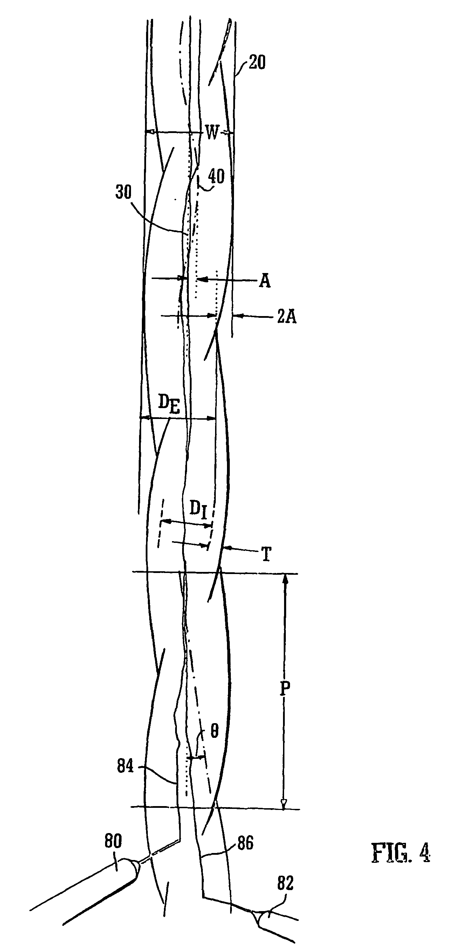

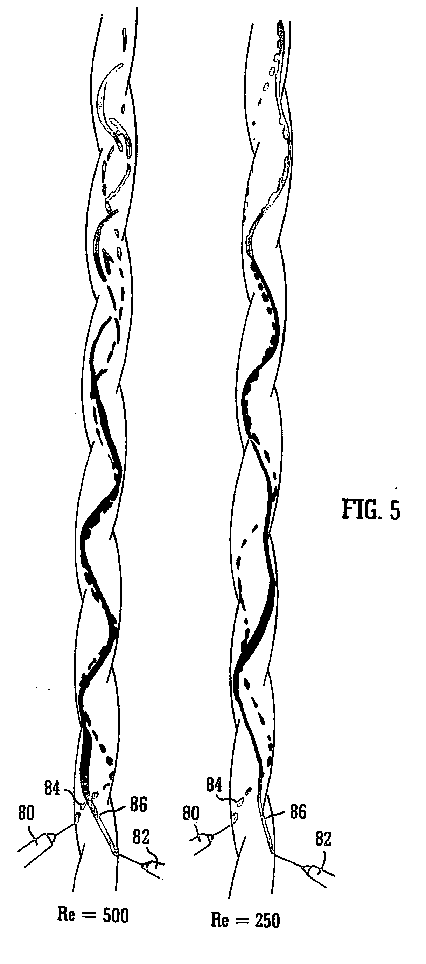

As mentioned above, making the fluid in the string flow in a swirl flow can avoid this problem, by preventing gas and water accumulating in the string. The characteristics of swirl flow, and a particular way of achieving swirl flow, will now be discussed with reference to Figures 4 and 5.

The tubing 1 shown in Figure 4 has a circular cross-section, an external diameter $D_E$, an internal diameter $D_I$ and a wall thickness T. The tubing is coiled into a helix of constant amplitude A (as measured from mean to extreme), constant pitch P, constant helix angle θ and a swept width W. The tubing 1 is contained in an imaginary envelope 20 which extends longitudinally and has a width equal to the swept width W of the helix. The envelope 20 may be regarded as having a central longitudinal axis 30, which may also be referred to as an axis of helical rotation. The illustrated tubing 1 has a straight axis 30, but it will be appreciated that in well production tubing the central axis will often have a large radius curvature (hence creating the U-bends). The tubing has a centre line 40 which follows a helical path about the central longitudinal axis 30.

It will be seen that the amplitude A is less than half the tubing internal diameter $D_I$. By keeping the amplitude below this size, the lateral space occupied by the tubing and the overall length of the tubing can be kept relatively small, whilst at the same time the helical configuration of the tubing promotes swirl flow of fluid along the tubing. This also provides a relatively wide lumen along the tubing, which allows instruments, apparatus and the like to be passed down the tubing.

## EXAMPLE 1

Experiments were carried out using polyvinyl chloride tubing with a circular cross-section. Referring to the parameters shown in Figure 4 the tubing had an external diameter $D_E$ of 12mm, an internal diameter $D_I$ of 8mm and a wall thickness T of 2mm. The tubing was coiled into a helix with a pitch P of 45mm and a helix angle θ of 8°. The amplitude A was established by resting the tubing between two straight edges and measuring the space between the straight edges. The amplitude was determined by subtracting the external diameter $D_E$ from the swept width W:

$$2A = W - D_E$$

So:

$$A = \frac{W - D_E}{2}$$

In this example the swept width W was 14 mm, so:

$$A = \frac{W - D_E}{2} = \frac{14-12}{2} = 1\ mm$$

As discussed earlier, "relative amplitude" $A_R$ is defined as:

$$A_R = \frac{A}{D_I}$$

In the case of this Example, therefore:

$$A_R = \frac{A}{D_I} = \frac{1}{8} = 0.125$$

Water was passed along the tube. In order to observe the flow characteristics, two needles 80 and 82 passing radially through the tube wall were used to inject visible dye into the flow. The injection sites were near to the central axis 30, i.e. at the "core" of the flow. One needle 80 injected red ink and the other needle 82 blue ink. It will be seen in Figure 4 that the ink filaments 84 and 86 intertwine, indicating that in the core there is swirl flow, i.e. flow which is generally helical. The experiment shown in Figure 4 was carried out at a Reynolds number $R_E$ of 500. In two further experiments, respectively using Reynolds numbers of 250 and 100, swirling core flow was also observed.

## EXAMPLE 2

The parameters for this Example were the same as in Example 1, except that the needles 80 and 82 were arranged to release the ink filaments 84 and 86 near to the wall of the tubing. Figure 5 shows the results of two experiments with near-wall ink release, with Reynolds numbers $R_E$ of 500 and 250 respectively. It will be seen that in both cases the ink filaments follow the helical tubing geometry, indicating near-wall swirl. Furthermore, mixing of the ink filaments with the water is promoted.

## EXAMPLE 3

In a separate study, the flow was compared in a straight 8 mm internal diameter tube with that in a helical 8 mm internal diameter tube, where the relative amplitude $A_R$ was 0.45. In both cases the Reynolds number was 500 and 0.2 ml indicator was injected as a bolus through a thin tube at the upstream end. The flows were photographed together with a digital clock to indicate elapsed time after the injection of indicator. The indicator front arrived earlier at the downstream end of the straight tube than of the helical tube and cleared later from the walls of the straight tube than from those of the helical tube.

Moreover, the indicator travelled in a more compact mass in the helical tube than in the straight tube. All these findings imply that there was mixing over the tube cross section and blunting of the velocity profile in the helical tube.

## EXAMPLE 4

The experiments of this Example involved a comparison of multi-phase flows in helical tubing with that in tubing having a centreline following a generally sinusoidal path in a single plane. In the case of the helical tubing (3 dimensional, i.e. 3D tubing), the internal diameter was 8 mm, the external diameter was 12 mm and the swept width was 17 mm, giving a relative amplitude of 0.3125. The pitch was 90 mm. In the case of the planar, wave-shaped tubing (2 dimensional, i.e. 2D tubing), the internal diameter was 8 mm, the external diameter was 12 mm, and the swept width, measured in the plane of the wave shape, was 17 mm. The pitch was 80 mm, not being significantly different from that of the 3D tubing case. The 2D tubing was held with its generally sinusoidal centreline in a vertical plane, in effect creating upwardly convex and concave U-bends.

Both the 3D and 2D tubes were about 400 mm in length, giving 4-5 pitches in each case. With both tubes, studies were performed with water flows of 450 and 900 ml per minute (Reynolds numbers of 1200 and 2400 respectively). A needle was used to introduce in all cases a flow of air at a rate of 3 ml per minute, i.e. 0.66% of the water flow in the 450 ml per minute case and 0.33% in the 900 ml per minute case. The air came' from a compressed air line and was injected into the tubes just upstream of the start of the respective 3D and 2D geometries.

In the case of the experiment with the 3D tubing at Reynolds number 1200, the air bubbles were about 2-3 mm in size and passed along the tube rapidly. At Reynolds number 2400, the bubbles were larger, about 5-7 mm but kept moving along the tube with no tendency to stick.

In the case of the 2D tubing at Reynolds numbers of 1200 and 2400, the bubbles were large, about 3-5 mm, and tended to stick in the upwardly convex curves (as viewed from outside the tubing).

The experiment shows that in a multi-phase flow the less dense fluid is carried along the 3D tubing, whereas in equivalent 2D tubing the less dense fluid tends to accumulate in the higher parts of the tubing.

Figure 3 shows a well having well production tubing in accordance with a preferred embodiment of the invention. This tubing is helical and the helical configuration causes swirl (or generally helical flow) along the tubing. As described previously, such flow has a centrifuge effect on the fluid in the pipe, such that denser material follows a helical path along the inside of the wall of the pipe, and less dense material flows along the centreline of the pipe. This tends to prevent pools of water from gathering in the upwardly concave U-bends of the well, thereby significantly reducing the chances of blockage. The tubing also tends to prevent pockets of gas from gathering in the upwardly convex U-bends, again reducing the chances of blockage.

A further problem which can arise in multiphase flows during hydrocarbon extraction is that of "slugging". This occurs when gas accumulates at the walls of the pipe, to such an extent as to block the flow. If the gas suddenly comes free from the walls, removing the blockage, then the flow will restart very suddenly, leading to impulse loads on the pipe and possible damage to the pipe and to ancillary equipment. Oil production platforms are routinely over-engineered to cope with such loads.

This problem can also be avoided by the use of swirl flow. As mentioned above, in multiphase swirl flow, the less dense fluids (such as gases) tend to the centre of the pipe, and so are kept away from the walls. They thus cannot accumulate to such an extent that they block the flow.

A similar advantage is obtained with the blood flow tubing mentioned above. As the air and oxygen bubbles tend to remain near the centre of the tubing, they are carried along with the rest of the flow, and do not accumulate and block the flow.

The fact that gas bubbles (or indeed any less dense fraction) will tend to the centre of the helical pipe provides further advantages with regard to reduction of the gas content of the flow.

In gas/liquid multiphase flow in a helical pipe, it has been found that the gas occupies a very small cross-sectional area at the centre of the pipe. In comparison to a straight pipe, the concentration of gas across the cross-section (usually referred to in the oil industry as the "cut") is reduced, and this reduction can be up to twenty or thirty percent. (It should be noted that the gas flow rate is the same in both pipes; the flow of the gas is faster in the helical pipe than in the straight pipe, to compensate for the smaller cross-sectional area of flow.)

This reduction in gas concentration can be highly beneficial with, for example, pumps. Pumps for liquids are not normally designed to cope with multiphase flow, and do not usually work well with high concentrations of gases. Reducing the concentration of gas in the flow by use of a helical pipe in this way will improve the efficiency of the pump.

A reduction in gas concentration can also be of benefit in other situations, where the flow must pass through a fitting which functions better with single-phase flow. A helical portion could be provided upstream of the fitting, to ensure that the fluid reaching the fitting is in a swirl flow condition, with the concentration of gas in the flow reduced.

A further beneficial effect obtained with multiphase swirl flow is a reduction in pressure drop; reductions of between ten and twenty percent, in comparison to the pressure drop in a straight tube, have been obtained in experiments with vertical pipes. A reduction in pressure drop would also allow an increased flow for the same pressure difference, and so would reduce the amount of energy required to pump a fluid.

Although the above description has concentrated particularly on the advantages which can be obtained in hydrocarbon extraction and in blood flow tubing, it will be appreciated that the tubing and piping of the invention can be applied to any multiphase flow, to obtain the advantages of swirl flow described above. In particular, the avoidance of gravitational effects such as phase separation is of particular relevance in the transport of slurries and suspensions of solids in liquids, as are frequently encountered in food processing, and in the transport of suspensions of powders in gas, as are frequently encountered in pharmaceutical production and processing.

**Claims**

1. Tubing or piping (1, 68) which is:

   well production tubing, such as risers, flow lines, and subsea and surface tubing; or
   blood flow tubing; or
   transport tubing or piping for transporting suspensions and/or slurries; or
   transport tubing or piping for transporting a suspension of powder in gas;

   the tubing or piping having features which induce swirl flow in a multiphase flow, in such a manner that denser components of the multiphase flow tend to the outer wall of the tubing or piping, and less dense components of the multiphase flow tend to the centre of the tubing or piping, as fluids flow along the pipe, wherein the centre line (40) of the tubing or piping follows a substantially helical path; **characterized in that** the amplitude (A) of the helix is less than or equal to one half of the internal diameter ($D_I$) of the tubing or piping.

2. Tubing or piping as claimed in claim 1, wherein the tubing or piping has a plurality of turns of the helix.

3. Tubing or piping as claimed in claim 1 or claim 2, wherein the helix has substantially the same amplitude (A) along the length of the tubing or piping.

4. Tubing or piping as claimed in any preceding claim, wherein the helix angle ($\theta$) is substantially the same along the length of the tubing or piping.

5. Tubing or piping as claimed in any preceding claim, wherein the tubing or piping has a substantially constant cross-sectional area along its length.

6. The use of tubing or piping as claimed in any preceding claim as well production tubing, such as risers, flow lines, and subsea and surface tubing.

7. The use of tubing or piping as claimed in any of claims 1 to 5 as blood flow tubing.

8. The use of tubing or piping as claimed in any of claims 1 to 5 to transport suspensions and/or slurries.

9. The use of tubing or piping as claimed in any of claims 1 to 5 to transport a suspension of powder in gas.

**Patentansprüche**

1. Verrohrung oder Verschlauchung (1, 68), welche besteht aus:

   Quellenförderungsverrohrung, wie Steigrohre, Fließleitungen, und Untermeeres- und Oberflächenverrohrung; oder
   Blutflussverschlauchung; oder
   Transportverrohrung oder -verschlauchung zum Transport von Aufschlämmungen und/oder Schlämmen; oder
   Transportverrohrung oder -verschlauchung zum Transport von Aufschlämmungen von Puder in Gasen;

   wobei die Verrohrung oder Verschlauchung Merkmale aufweist, die Drallströmung in einer Mehrphasenströmung induzieren, auf solch eine Art und Weise, dass dichtere Bestandteile der Mehrphasenströmung zur Außenwand der Verrohrung oder Verschlauchung tendieren, und weniger dichte Bestandteile der Mehrphasenströmung zum Zentrum der Verrohrung oder Verschlauchung tendieren, während Flüssigkeiten entlang der Rohrleitung strömen, wobei die Mittelachse (40) der Verrohrung oder Verschlauchung einer im Wesentlichen schraubenförmigen Bahnkurve folgt; **gekennzeichnet dadurch dass** die Amplitude (A) der Schraubenlinie kleiner oder gleich einer Hälfte des internen Durchmessers ($D_r$) der Verrohrung oder Verschlauchung ist.

2. Verrohrung oder Verschlauchung gemäß Anspruch 1, wobei die Verrohrung oder Verschlauchung eine Vielzahl von Windungen der Schraubenlinie aufweist.

3. Verrohrung oder Verschlauchung gemäß einem der Ansprüche 1 oder 2, wobei die Schraubenlinie im Wesentlichen die gleiche Amplitude (A) entlang der Länge der Verrohrung oder Verschlauchung aufweist.

4. Verrohrung oder Verschlauchung gemäß einem der vorhergehenden Ansprüche, wobei der Schrägungs- bzw. Steigungswinkel ($\theta$) im Wesentlichen der gleiche entlang der Länge der Verrohrung oder Verschlauchung ist.

5. Verrohrung oder Verschlauchung gemäß einem der vorhergehenden Ansprüche, wobei die Verrohrung oder Verechlauchung entlang ihrer Länge eine im Wesentlichen konstante Querschnittsfläche aufweist.

6. Die Verwendung der Verrohrung oder Verschlauchung gemäß einem der vorhergehenden Ansprüche sowie auch von Quellenförderungsverrohrung, wie Steigrohre, Fließleitungen, und Untermeeres- und Oberflächenverrohrung.

7. Die Verwendung der Verrohrung oder Verschlauchung gemäß einem der Ansprüche 1 bis 5 als Blutflussverschlauchung.

8. Die Verwendung der Verrohrung oder Verschlauchung gemäß einem der Ansprüche 1 bis 5, um Aufschlämmungen und/oder Schlämme zu transportieren.

9. Die Verwendung der Verrohrung oder verschlauchung gemäß einem der Ansprüche 1 bis 5, um eine Aufschlämmung von Puder in Gasen zu transportieren.

**Revendications**

1. Tube ou canalisation (1, 68) qui est :

une colonne de production de puits, telle que des tubes prolongateurs, des conduites de collecte et des tubes sous-marins et de surface ; ou des tubes d'écoulement du sang ; ou des tubes ou des canalisations de transport de suspensions et/ou de boues ; ou des tubes ou des canalisations de transport d'une suspension de poudre dans du gaz ; le tube ou la canalisation ayant des caractéristiques qui induisent un courant turbulent dans un courant multiphasé de façon à ce que des constituants plus denses du courant multiphasé tendent à aller vers la paroi extérieure du tube ou de la canalisation, et des constituants moins dense du courant multiphasé tendent à aller vers le centre du tube ou de la canalisation alors que des fluides s'écoulent le long du conduit, la ligne (40) centrale du conduit ou de la canalisation suivant un trajet sensiblement hélicoïdal ; **caractérisé en ce que** l'amplitude (A) de l'hélice est inférieure ou égale à une moitié du diamètre ($D_r$) intérieur du tube ou de la canalisation.

2. Tube ou canalisation suivant la revendication 1, dans lequel le tube ou la canalisation a une pluralité de tours de l'hélice.

3. Tube ou canalisation suivant la revendication 1 ou 2, dans lequel l'hélice a sensiblement la même amplitude (A) sur la longueur du tube ou de la canalisation.

4. Tube ou canalisation suivant l'une quelconque des revendications précédentes, dans lequel l'angle (θ) de l'hélice est sensiblement le même sur la longueur du tube ou de la canalisation.

5. Tube ou canalisation suivant l'une quelconque des revendications précédentes, dans lequel le tube ou la canalisation a une surface de section transversale sensiblement constante sur sa longueur.

6. L'utilisation de tube ou de canalisation, tel que revendiqué à l'une quelconque des revendications précédentes, ainsi que tube de production de puits, tel que tubes prolongateurs, conduites de collecte et tube sous-marin et de surface.

7. L'utilisation de tube ou de canalisation suivant l'une quelconque des revendications 1 à 5, comme tube d'écoulement du sang.

8. L'utilisation de tube ou de canalisation suivant l'une quelconque des revendications 1 à 5, pour transporter des suspensions et/ou des boues.

9. L'utilisation de tube ou de canalisation suivant l'une quelconque des revendications 1 à 5 pour transporter une suspension de poudre dans du gaz.

FIG. 1

**FIG. 2**

**FIG. 3**

EP 1 611 385 B1

FIG. 4

FIG. 5

Re = 500        Re = 250

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0038591 A